# EUROPÄISCHE PATENTSCHRIFT

(11) **EP 1 761 757 B1**
(45) Veröffentlichungstag und Bekanntmachung des Hinweises auf die Patenterteilung: **02.03.2016**
(21) Anmeldenummer: 05751698.1
(22) Anmeldetag: 03.06.2005
(51) Int. Cl.: B01L 3/00, G01N 33/49

(54) **VORRICHTUNG ZUR AUFNAHME VON BLUT UND ABTRENNUNG VON BLUTBESTANDTEILEN, SOWIE VERWENDUNGEN DER VORRICTUNG**
DEVICE FOR COLLECTING BLOOD AND SEPARATING BLOOD CONSTITUENTS, AND USE OF SAID DEVICE
DISPOSITIF POUR RECUEILLIR DU SANG ET SEPARER DES CONSTITUANTS SANGUINS, ET UTILISATION DUDIT DISPOSITIF

(30) Priorität: 04.06.2004 DE 102004027422
(43) Veröffentlichungstag der Anmeldung: 14.03.2007
(73) Patentinhaber: Boehringer Ingelheim microParts GmbH, 44227 Dortmund (DE)
(72) Erfinder: BLANKENSTEIN, Gert, 44141 Dortmund (DE); PETERS, Ralf-Peter, 51467 Bergisch-Gladbach (DE)
(74) Vertreter: Von Rohr Patentanwälte Partnerschaft mbB
(86) Internationale Anmeldenummer: PCT/EP2005/005982
(87) Internationale Veröffentlichungsnummer: WO 2005/119211

(56) Entgegenhaltungen:
- EP-A- 0 348 006
- EP-A- 0 977 032
- DE-A1- 10 142 788
- US-A- 4 883 688
- US-A- 6 156 270
- US-B1- 6 391 265

## Beschreibung

Die vorliegende Erfindung betrifft eine Vorrichtung zur Aufnahme von Blut und Abtrennung von Blutbestandteilen, wie Blutplasma, als Probenflüssigkeit gemäß dem Oberbegriff des Anspruchs 1 sowie Verwendung der Vorrichtung zur Bestimmung von einem Blutbestandteil oder mehreren Blutbestandteilen.

Die vorliegende Erfindung befasst sich mit mikrofluidischen Systemen bzw. Vorrichtungen. Die nachfolgenden Ausführungen beziehen sich auf Vorrichtungen, bei denen Kapillarkräfte wirken und insbesondere für die Funktion entscheidend sind.

Aus der EP 1 013 341 A2, die den Ausgangspunkt der vorliegenden Erfindung bildet, ist eine Vorrichtung zum Abtrennen von flüssigen Komponenten bekannt, wobei beispielsweise Blutplasma als Probenflüssigkeit in einem Filter oder einer Membran von Blut abgetrennt und durch Kapillarkräfte in einen Sammelraum bzw. Kanal zur Diagnostik aufgenommen wird. Der Kanal weist einen im wesentlichen rechteckigen Querschnitt auf. Er ist in einem Träger durch eine entsprechende Ausnehmung gebildet, die oberseitig abgedeckt ist. Weiter weist der Kanal keilförmige Kerben als Leitelemente auf, um ein vollständiges Füllen des im Querschnitt zunehmenden Kanals mit der Probenflüssigkeit sicherzustellen. Die vorgeschlagene Kartalgestaltung gestattet jedoch noch kein optimales Füllen mit der Probenflüssigkeit.

Aus der US 4,906,439 A und der WO 01/24931 A1 sind jeweils Vorrichtungen zur Abtrennung von Blutplasma aus Blut bekannt, wobei eine Vielzahl von nutartigen bzw. kapillarartigen Einzelkanälen zur Aufnahme und Ableitung des Blutplasmas vorgesehen sind. Hier tritt der Nachteil auf, dass sich die Kanäle unterschiedlich schnell oder gar nicht mit der Probenflüssigkeit in Form von Blutplasma füllen. Dementsprechend kann keine einheitliche Flüssigkeitsfront erreicht werden. Dies ist hinsichtlich der Diagnostik nachteilig, da keine definierte Menge gleichzeitig zur Verfügung steht oder beispielsweise nicht gleichzeitig Trockenchemikalien oder dergleichen in dem gewünschten bzw. erforderlichen Maß von der Probenflüssigkeit gelöst werden können.

Die EP 0 977 032 A1 betrifft eine Vorrichtung zur Aufnahme und Abtrennung von Blutbestandteilen. Die Vorrichtung weist eine Kapillare mit einer Einfüllöffnung auf. Die Kapillare ist mit zwei hydrophilen und einem hydrophoben Bereich versehen. Der erste hydrophile Bereich nimmt die Flüssigkeit aus der Einfüllöffnung auf, wobei eine Membrane vor dem ersten hydrophilen Bereich angeordnet sein kann. Der zweite hydrophile Bereich ist mit einem Reagenz gefüllt. Der hydrophobe Bereich trennt die hydrophilen Bereiche voneinander und ist mit einer quer zum Kanal angeordneten Entlüftung versehen. Durch externe Kräfte wird die Probenflüssigkeit aus dem ersten hydrophilen Bereich in den zweiten hydrophilen Bereich transferiert.

Die EP 0 348 006 A2 offenbart eine Vorrichtung, die zur Aufnahme und Abtrennung von Blutbestandteilen geeignet ist. Die Vorrichtung weist einen seitenwandfreien Kanal auf, der zwischen einem Träger, eine Abdeckung und Abstandshaltern gebildet wird. In der Abdeckung ist eine Öffnung zur fluidischen Verbindung mit dem Kanal ausgebildet, die ein Füllelement zur Füllung des Kanals aufnehmen kann. Alternativ können Leitelemente in der Öffnung angeordnet sein.

In der US 6,165,270 A wird eine Vorrichtung, die zur Aufnahme und Abtrennung von Blutbestandteilen geeignet ist, offenbart. Die Vorrichtung weist eine Zuführung mit darin Integrierter Trenneinrichtung, ein daran anschließende seitenwandfreier Kanal mit Einlassbereich und eine Entlüftungseinrichtung auf. Der Entlüftungskanal ist als Ausnehmung ausgebildet und begrenzt den Kanal.

Die DE 101 42 788 A1 betrifft eine Analysevoreichtung zur Analyse von Makromolekolen oder Gewebeschnitten. Die Vorrichtung weist einen Träger, eine Abdeckung und Abstandshalter zwischen Träger und Abdeckung auf, so dass ein Kapillarkanal gebildet wird. Der Kanal kann durch eine Öffnung befüllt werden, wobei Luft zwischen Träger und Abdeckung zur Seite entweichen kann. Am Rand der Vorrichtung kann ein Ansatz vorgesehen sein, der den Abstand zwischen Abdeckung und Träger vergrößert und als Kapillarstop wirkt.

Der vorliegenden Erfindung liegt die Aufgabe zugrunde, eine Vorrichtung zur Aufnahme von Blut und Abtrennung von Blutbestandteilen, wie Blutplasma, als Probenflüssigkeit sowie Verwendung der Vorrichtung zur einfachen und schnellen Bestimmung von Plasmabestandteilen anzugeben, wobei ein optimiertes Abtrennen der Blutbestandteile und Füllen des Kanals mit Probenflüssigkeit ermöglicht wird und vorzugsweise die diagnostischen bzw. Untersuchungsmöglichkeiten verbessert werden.

Die obige Aufgabe wird durch eine Vorrichtung gemäß Anspruch 1 oder eine Verwendung gemäß Anspruch 18 gelöst. Vorteilhafte Weiterbildungen sind Gegenstand der Unteransprüche.

Ein Aspekt liegt darin, die Probenflüssigkeit in dem sich unmittelbar an die Trenneinrichtung abströmseitig anschließenden Einlaßbereich des Kanals quer zur Hauptfüllrichtung des Kanals mit Probenflüssigkeit und/oder quer zur Langserstreckung des Kanals zu entlüften.

Die vorschlagsgemäße Entlüftung gestattet ein optimales, gleichmäßiges Füllen des Kanals, insbesondere im Einlaßbereich, und verhindert unerwünschte Lufteinflüsse.

Die vorschlagsgemäße Entlüftung gestattet außerdem eine besonders effiziente Bluttrennung. Wenn sich Lufteinschlüsse an der Trenneinrichtung auf der Abströmseite bilden, blockieren diese. Lufteinschlüsse die Ableitung von Probenflüssigkeit aus der Trenneinrichtung. Entsprechend können in diesem Fall nur diejenigen Bereiche der Trenneinrichtung die Abtrennung der Probenflüssigkeit vornehmen, die nicht durch Lufteinschlüsse blockiert sind. Diese Lufteinschlüsse können durch die Trenneinrichtung, insbesondere eine gasdurchlässige Membran oder ein sonstiges Filterelement, nicht entweichen, da das aufzutrennende Blut die Trenneinrichtung auf der Zuführseite üblicherweise vollständig bedeckt und dementsprechend eine Entlüftung blockiert. Die vorschlagsgemäße Entlüftung gestattet eine Ableitung und damit Verhinderung derartiger Lufteinschlüsse auf der Abströmseite der Trenneinrichtung, so daß die Trenneinrichtung optimal zur Bluttrennung genutzt wird und dementsprechend eine effektive und schnelle Bluttrennung erfolgt.

Die Entlüftungseinrichtung ist vorzugsweise zur Entlüftung der Probenflüssigkeit unmittelbar an der Abströmseite der Trenneinrichtung ausgebildet. Insbesondere ist die Entlüftungseinrichtung zur Entlüftung der Probenflüssigkeit im Einlaßbereich in der Haupterstreckungsebene des Kanals und/oder entlang einer Flachseite der Trenneinrichtung und/oder quer zur Strömungseinrichtung der Probenflüssigkeit durch die Trenneinrichtung ausgebildet. So wird eine wirksame Entlüftung des Einlaßbereichs des Kanals ermöglicht.

Vorzugsweise weist die Entlüftungseinrichtung mindestens einen quer zur Hauptfüllrichtung des Kanals verlaufenden Querkanal im Einlaßbereich auf. Es können auch mehrere parallel verlaufende Querkanäle vorgesehen sein. Dies gestattet auf einfache Weise eine wirksame Entlüftung.

Alternativ oder zusätzlich kann der Kanal zumindest im Einlaßbereich - insbesondere entlang der Längs- bzw. Schmalseiten - seitlich offen ausgebildet sein, um die vorschlagsgemäße Entlüftung im Einlaßbereich zu realisieren.

Insbesondere ist der Kanal dann an gegenüberliegenden Längs- oder Schmalseiten des Einlaßbereiches offen ausgebildet, um eine besonders wirksame Entlüftung der Probenflüssigkeit im Einlaßbereich zu erreichen und Lufteinschlüsse an der Abströmseite der Trenneinrichtung zu verhindern.

Die Probenflüssigkeit wird durch Kapillarkräfte in dem Kanal aufgenommen, der vorzugsweise zumindest an einer Schmalseite oder Längsseite offen ausgebildet ist, so daß ein seitlicher Flüssigkeitsstopp für die Probenflüssigkeit im Kanal gebildet wird und die Probenflüssigkeit seitenwandfrei im Kanal führbar ist. Insbesondere schließt sich hierbei eine Ausnehmung seitlich an die offene Seite des Kanals an.

Die seitlich offene Ausbildung des Kanals gestattet eine verbesserte, insbesondere optimale Entlüftung beim Füllen des Kanals mit Probenflüssigkeit, insbesondere sowohl im Einlaßbereich als auch in sonstigen Kanalbereichen.

Durch die seitenwandfreie Führung kann auf einfache Weise ein Vorschießen der Probenflüssigkeit - also schnelleres Füllen des Kanals - im Bereich einer ansonsten vorhandenen Seitenwand verhindert werden. Dies ermöglicht eine Vergleichmäßigung der Füllgeschwindigkeit über den gesamten Kanalquerschnitt, so daß eine zumindest im wesentlichen gleichmäßige bzw. gradlinige Flüssigkeitsfront beim Füllen des Kanals erreichbar ist. Dies gestattet außerdem ein optimales, gleichmäßiges Füllen des Kanals und verhindert unerwünschte Lufteinschlüsse. Jedoch können auch andere Maßnahmen zum Verhindern des Vorschießens von Probenflüssigkeit entlang der Schmal- bzw. Längsseiten des Kanals, wie eine entsprechend modifizierte, beispielsweise mit Erhebungen versehene Seitenwandung, vorgesehen sein.

Außerdem gestattet die seitenwandfrei gehaltene bzw. geführte Oberfläche der Probenflüssigkeit eine unmittelbare Untersuchung der Probenflüssigkeit, insbesondere durch Einkopplung von Licht, ohne eine ansonsten vorhandene Seitenwand o. dgl.

Eine vorschlagsgemäße Verwendung der Vorrichtung zeichnet sich dadurch aus, daß unmittelbar eine Lysierung mittels einer ersten Chemikalie und direkt anschließend eine Bestimmung des intrazellulären Parameters mittels einer zweiten Chemikalie erfolgt. Dies gestattet bei einfachem und kompaktem Aufbau eine schnelle und preisgünstige Analyse bzw. Bestimmung des Parameters.

Eine weitere vorschlagsgemäße Verwendung der Vorrichtung zeichnet sich dadurch aus, daß unmittelbar nach dem Zurückhalten bzw. Abtrennen von Blutzellen direkt eine Bestimmung eines Bestandteils bzw. Parameters des Blutplasmas mittels einer Chemikalie oder mehreren Chemikalien erfolgt. Dies gestattet bei einfachem und kompaktem Aufbau eine schnelle und preisgünstige Analyse bzw. Bestimmung des Parameters.

Weitere Vorteile, Merkmale, Eigenschaften und Aspekte der vorliegenden Erfindung ergeben sich aus den Ansprüchen und der folgenden Beschreibung bevorzugter Ausführungsformen anhand der Zeichnung. Es zeigt:
- Fig. 1: einen schematischen Schnitt einer vorschlagsgemäßen Vorrichtung gemäß einer ersten Ausführungsform, welche nicht beansprucht wird;
- Fig. 2a: eine schematische Draufsicht eines Trägers der gefüllten Vorrichtung gemäß Fig. 1;
- Fig. 2b: eine ausschnittsweise Vergrößerung von Fig. 2a;
- Fig. 3: einen schematischen Schnitt der Vorrichtung entlang Linie III-III gemäß Fig. 2a;
- Fig. 4: einen schematischen Längsschnitt der Vorrichtung entlang Linie IV-IV gemäß Fig. 2a;
- Fig. 5: eine schematische Draufsicht eines Trägers einer vorschlagsgemäßen Vorrichtung gemäß einer ebenfalls nicht beanspruchten zweiten Ausführungsform;
- Fig. 6: eine ausschnittsweise schematische Draufsicht eines Trägers einer vorschlagsgemäßen Vorrichtung gemäß einer ebenfalls nicht beanspruchten dritten Ausführungsform;
- Fig. 7: einen ausschnittsweisen, schematischen Schnitt der Vorrichtung entlang Linie VII-VII gemäß Fig. 6;
- Fig. 8: einen schematischen Längsschnitt einer vorschlagsgemäßen Vorrichtung gemäß einer beanspruchten vierten Ausführungsform;
- Fig. 9a: einen schematischen Schnitt einer vorschlagsgemäßen Vorrichtung gemäß einer ebenfalls nicht beanspruchten fünften Ausführungsform;
- Fig. 9b: eine ausschnittsweise Vergrößerung von Fig. 9a;
- Fig. 10: einen schematischen Schnitt einer vorschlagsgemäßen Vorrichtung gemäß einer ebenfalls nicht beanspruchten sechsten Ausführungsform;
- Fig. 11: eine schematische Draufsicht eines Trägers einer vorschlagsgemäßen Vorrichtung gemäß einer beanspruchten siebten Ausführungsform; und
- Fig. 12: einen schematischen Schnitt der Vorrichtung entlang Linie XII - XII gemäß Fig. 11.

In den Figuren werden für gleiche oder ähnliche Teile dieselben Bezugszeichen verwendet, wobei entsprechende oder vergleichbare Eigenschaften und Vorteile erreicht werden, auch wenn eine wiederholte Beschreibung weggelassen ist.

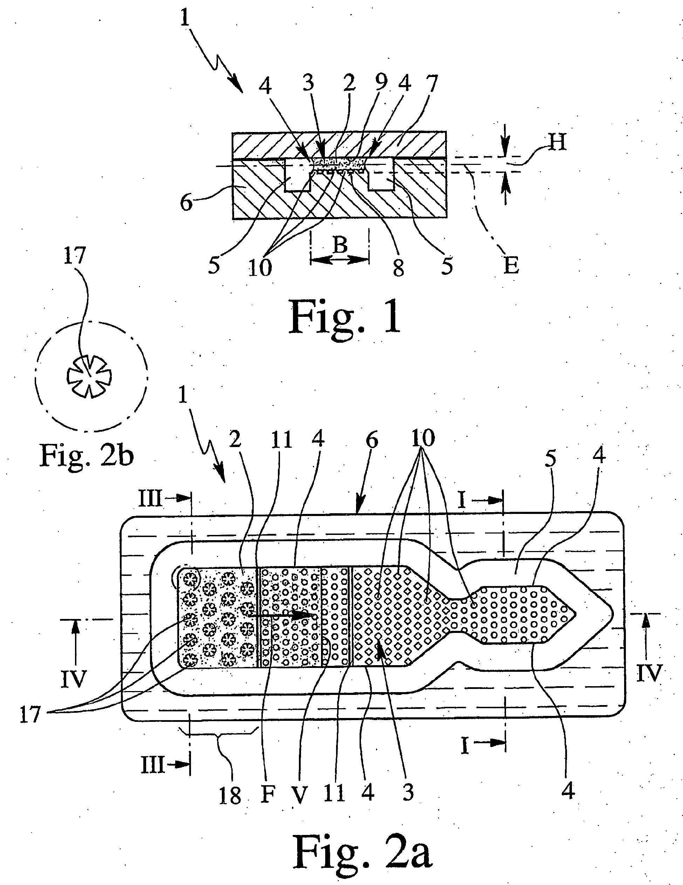

Fig. 1 zeigt in einem schematischen Schnitt eine erste nicht beanspruchte Ausführungsform einer vorschlagsgemäßen Vorrichtung 1 zur Aufnahme und/oder Diagnostik einer Probenflüssigkeit 2, insbesondere Blutplasma o. dgl.

Die Vorrichtung 1 weist einen die Probenflüssigkeit 2 durch Kapillarkräfte aufnehmenden Kanal 3 auf. Der Kanal 3 ist vorzugsweise zumindest an einer Schmalseite bzw. Längsseite 4, beim Darstellungsbeispiel auf beiden Schmal- bzw. Längsseiten 4, offen ausgebildet, wie in Fig. 1 angedeutet.

Seitlich schließt sich an die offenen Seiten 4 eine Ausnehmung 5 an, die beim Darstellungsbeispiel vorzugsweise nut- bzw. grabenartig ausgebildet ist. So wird ein seitlicher Flüssigkeitsstopp für die Pröbenflüssigkeit 2 - also ein durch Kapillarkräfte nicht überwindbares Strömungshindernis - im Kanal 3 gebildet und ist die Probenflüssigkeit 2 seitenwandfrei entlang der offenen Seiten 4 im Kanal 3 führbar.

Beim Darstellungsbeispiel weist die Vorrichtung 1 einen Träger 6 und eine zugeordnete Abdeckung 7 auf, zwischen denen der Kanal 3 und die Ausnehmung 5 gebildet sind. Bedarfsweise ist lediglich der Träger 6 zur Bildung der erforderlichen Strukturen ausgenommen und die Abdeckung 7 eben, vorzugsweise zumindest im wesentlichen ausnehmungsfrei, ausgebildet. Jedoch kann dies auch umgekehrt sein. Bedarfsweise können aber auch sowohl der Träger 6 als auch die Abdeckung 7 ausgenommen und/oder mit Vorsprüngen zur Bildung der gewünschten Strukturen und ggf. zur Aufnahme von nicht dargestellten Chemikalien, Reagenzien, Untersuchungseinrichtungen o. dgl. ausgebildet sein.

Die Ausnehmung 5 schließt sich vorzugsweise scharfkantig an den Kanal 3 an, wie in Fig. 1 angedeutet. Beim Darstellungsbeispiel ist die Ausnehmung 5 nur im Träger 6 gebildet, erstreckt sich bei der Darstellung gemäß Fig. 1 also im wesentlichen nur nach unten bezüglich einer seitlichen Projektion des Kanals 3. Die Ausnehmung 5 kann sich jedoch wahlweise auch nach oben oder auf beide Seiten der seitlichen Projektion des Kanals 3 - also insbesondere nach oben und nach unten - erstrecken.

Die im Querschnitt vorzugsweise rechteckige Ausnehmung 5 führt zu einer derartigen, insbesondere stufigen bzw. plötzlichen Querschnittsvergrößerung, daß sich die Kapillarkräfte derartig verringern, daß der genannte Flüssigkeitsstopp für die Probenflüssigkeit 2 im Übergang vom Kanal 3 zur Ausnehmung 5 hin gebildet wird, wie in Fig. 1 angedeutet.

Der Kanal 3 wird vorzugsweise von nur zwei gegenüberliegenden, insbesondere im wesentlichen ebenen Flächen bzw. Flachseiten 8 und 9, die beim Darstellungsbeispiel durch den Träger 6 bzw. die Abdeckung 7 gebildet sind und parallel verlaufen, begrenzt bzw. gebildet. Bedarfsweise kann daher die Ausnehmung 5 auch ganz entfallen und der Kanal 3 beispielsweise durch zwei geeignete Stege o. dgl. mit geeignetem Abstand zur Erzeugung der gewünschten Kapillarkräfte gebildet sein.

Fig. 2a zeigt in einer schematischen Draufsicht den Träger 6 der Vorrichtung 1 ohne Abdeckung 7, jedoch mit teilweiser Füllung durch die Probenflüssigkeit 2 bis zur Flüssigkeitsfront V.

Die Ausnehmung 5 erstreckt sich beim Darstellungsbeispiel entlang der offenen Seite(n) 4 des Kanals 3, vorzugsweise zumindest entlang gegenüberliegender, offener Längsseiten 4. Weiter ist beim Darstellungsbeispiel der Kanal 3 allseitig seitlich offen ausgebildet und die Ausnehmung 5 dementsprechend umlaufend ausgebildet. Der Kanal 3 ist also allseitig von der Ausnehmung 5 umgeben.

Vorzugsweise schließt sich die Ausnehmung 5 an diejenigen Schmalseiten bzw. Längsseiten 4 des Kanals 3 an, die sich zumindest im wesentlichen parallel zur Hauptfüllrichtung F des Kanals 3 mit Probenflüssigkeit 2 (Hauptströmungsrichtung der Probenflüssigkeit 2 im Kanal 3), wie in Fig. 2a angedeutet, erstrecken. Folglich verläuft die Ausnehmung 5 vorzugsweise zumindest abschnittsweise parallel zur Hauptfüllrichtung F.

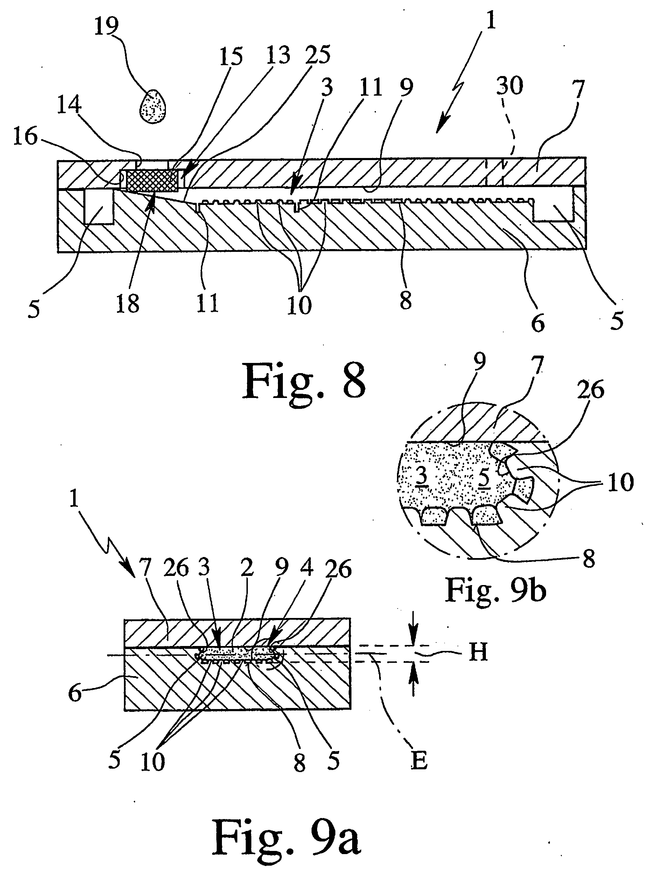

Gemäß einer anderen, später anhand von Fig. 9a erläuterten Variante ist es auch möglich, daß sich die Ausnehmung 5 mit der Probenflüssigkeit 2 oder einer anderen, sich mit der Probenflüssigkeit 2 insbesondere nicht mischenden Flüssigkeit, wie Öl oder dergleichen, füllt. In diesem Fall ist die Ausnehmung 5 oder die Seitenwandung des Kanals 3 jedoch derart gestaltet, daß deren Füllgeschwindigkeit maximal so groß wie die Füllgeschwindigkeit des Kanals 3 ist, um ein möglichst gleichmäßiges Füllen mit Probenflüssigkeit 2 zu erreichen. Die Füllgeschwindigkeiten beziehen sich dabei jeweils auf das Füllen bzw. Voranschreiten der Flüssigkeitsfront V in die Hauptfüllrichtung F.

Alternativ kann die Ausnehmung 5 vor Einleiten der Probenflüssigkeit 2 auch nur mit der anderen Flüssigkeit gespült werden.

Der Kanal 3 weist vorzugsweise einen im wesentlichen rechteckigen und/oder flachen Querschnitt, insbesondere quer zur Hauptfüllrichtung F, auf.

Die in Fig. 1 angedeutete Höhe H des Kanals 3 - also der Abstand der den Kanal 3 begrenzenden, vorzugsweise parallelen Flächen 8 und 9 - beträgt maximal 2000 µm, vorzugsweise höchstens 500 µm, insbesondere etwa 50 bis 200 µm. Die Ausnehmung 5 führt vorzugsweise zu einer stufigen bzw. plötzlichen Vergrößerung der Höhe H und dadurch zur Bildung des gewünschten Flüssigkeitstopps. Insbesondere ist die Höhe H der Ausnehmung 5 mindestens doppelt so groß wie die Höhe H des Kanals 3.

Die Breite B des Kanals 3 beträgt vorzugsweise etwa 100 bis 5000 µm, insbesondere etwa 200 bis 4000 µm.

Die Höhe H des Kanals 3 ist wesentlich geringer, insbesondere mindestens um den Faktor 5 oder 10, als die Breite B des Kanals 3.

Das Aufnahmevolumen des Kanals 3 beträgt vorzugsweise weniger als 1 ml, insbesondere weniger als 100 µl, besonders bevorzugt maximal 10 µl.

Die Vorrichtung 1 bildet also ein mikrofluidisches System. Insbesondere dient die Vorrichtung 1 der mikrofluidischen Diagnostik für medizinische oder nicht-medizinische Zwecke bzw. sonstige Untersuchungen.

Der Kanal 3 und damit dessen Hauptfüllrichtung F und Haupterstreckungsebene E verlaufen in Gebrauchslage vorzugsweise zumindest im wesentlichen horizontal. Je nach Verwendungszweck oder konstruktiver Lösung ist jedoch auch eine andere Ausrichtung möglich, zumal die Aufnahme bzw. das Füllen des Kanals 3 mit Probenflüssigkeit 2 vorzugsweise zumindest primär nur durch Kapillarkräfte bestimmt bzw. bewirkt wird. So kann die Hauptfüllrichtung F beispielsweise horizontal oder geneigt verlaufen, während die Haupterstreckungsebene E beispielsweise vertikal verläuft, so daß der Kanal 3 also hochkant ausgerichtet ist.

Der Kanal 3 bildet vorzugsweise mindestens einen Speicher für die Probenflüssigkeit 2, insbesondere zur Diagnostik. Ggf. kann der Kanal 3 eine nicht dargestellte Chemikalie, insbesondere eine Trockenchemikalie o. dgl., enthalten. Jedoch können Untersuchungen der Probenflüssigkeit 2 auch auf sonstige Weise vorgenommen werden.

Beim Darstellungsbeispiel weist der Kanal 3 mindestens ein Leitelement zur Beeinflussung, insbesondere Vergleichmäßigung, des Füllens mit der Probenflüssigkeit 2 auf.

Gemäß einer Ausbildungsvariante weist der Kanal 3 vorzugsweise regelmäßig verteilte Erhöhungen 10 als Leitelemente auf. Diese sind insbesondere in Reihen quer, vorzugsweise senkrecht, oder längs zur Hauptfüllrichtung F, insbesondere abwechselnd quer versetzt, angeordnet. Die Erhöhungen 10 sind die Reihen in Hauptfüllrichtung F versetzt. So kann erreicht werden, daß die Probenflüssigkeit 2 den Kanal 3 reihenweise - also Reihe für Reihe - füllt und dadurch mit einer im wesentlichen gradlinigen Flüssigkeitsfront V in Hauptfüllrichtung F fortschreitet.

Bedarfsweise kann die Flächendichte, der Abstand und/oder die Größe der Erhöhungen 10 variieren, insbesondere in Abhängigkeit von der jeweiligen Entfernung zu einem in Fig. 1 und 2 nicht dargestellten Einlaß für die Probenflüssigkeit 2 in den Kanal 3.

Die Erhöhungen 10 sind vorzugsweise steg-, höcker- oder säulenartig, insbesondere mit runder oder polygonaler Grundfläche, ausgebildet. Statt dessen können aber auch Vertiefungen vorgesehen sein.

Alternativ oder zusätzlich kann der Kanal 3 mindestens einen Graben 11 oder einen Steg als Leitelement aufweisen, der quer oder längs zur Hauptfüllrichtung F des Kanals 3 verläuft. Der vorzugsweise vorgesehene nutartige, im Querschnitt insbesondere rechteckige oder halbrunde Graben 11 weist eine wesentlich geringere Tiefe als die Ausnehmung 5 auf und bildet daher einen nur temporären Flüssigkeitsstopp zur Vergleichmäßigung der Flüssigkeitsfront V. So kann erreicht werden, daß die Probenflüssigkeit 2 erst nach Füllen des Kanals 3 über seinen gesamten Querschnitt den Graben 11 und anschließend den nachfolgenden Kanalbereich füllt.

Hervorzuheben ist, daß durch die Kombination der seitenwandlosen Führung der Probenflüssigkeit 2 und der Leitelemente ein hochgradig gleichmäßiges Füllen des Kanals 3 durch Kapillarkräfte mit zumindest im wesentlichen gradliniger bzw. senkrecht zur Hauptfüllrichtung F verlaufender Flüssigkeitsfront V erreichbar ist.

Alternativ kann der Kanal 3 und/oder ein davon gebildeter Speicher, Sammelraum, Sammelbereich o. dgl. auch zumindest im wesentlichen glatt bzw. eben, also insbesondere ohne Leitelemente, ausgebildet sein.

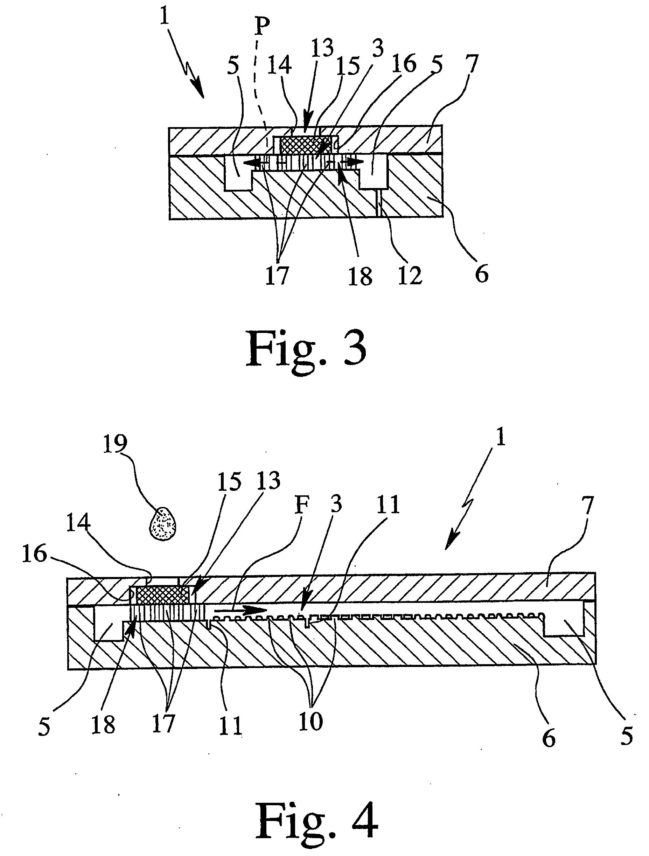

Fig. 3 zeigt einen weiteren schematischen Schnitt der Vorrichtung 1 mit der Abdeckung 7 entlang Linie III-III gemäß Fig. 2a.

Die Vorrichtung 1 weist mindestens einen dem Kanal 3 zugeordneten Entlüftungskanal 12 auf, der beim Ausführungsbeispiel nicht unmittelbar an den Kanal 3, sondern an die Ausnehmung 5 angeschlossen ist. So ist kein zusätzlicher Flüssigkeitsstopp für die Entlüftung 12, um ein Austreten von Probenflüssigkeit 2 durch die Entlüftung 12 zu verhindern, erforderlich. Die vorzugsweise allseitig seitlich offene Ausbildung des Kanals 3 gestattet ein optimales Entlüften beim Füllen des Kanals 3 mit der Probenflüssigkeit 2, so daß unerwünschte Lufteinschlüsse sicher vermieden werden können.

Die Probenflüssigkeit 2 ist dem Kanal 3 vorzugsweise senkrecht zur Kanalerstreckung E, insbesondere in Gebrauchslage vertikal, zuführbar.

Die Vorrichtung 1 weist eine Zuführeinrichtung 13 zur Aufnahme von Blut 19, auf, wie in Fig. 4 angedeutet. Beim Darstellungsbeispiel ist die Zuführeinrichtung 13 durch eine Öffnung, insbesondere Durchbrechung 14, in der Abdeckung 7 gebildet. Die Vorrichtung 1 weist ferner eine sich anschließende Trenneinrichtung 15, wie einen Filter, eine Membran o. dgl., zur Abtrennung von Blutplasma als Probenflüssigkeit 2 auf.

Die Trenneinrichtung 15 ist beim Darstellungsbeispiel in eine zum Träger 6 hin offene Aussparung 16 in der Abdeckung 7 eingesetzt und deckt die Durchbrechung 14 ab.

Vorzugsweise ist die Trenneinrichtung 15 mit der Abdeckung 7 fest verbunden, beispielsweise verschweißt, verklebt oder von dieser kraft- oder formflüssig gehalten.

Die Trenneinrichtung 15 steht - beim Darstellungsbeispiel mit einer Flachseite - unmittelbar mit dem Kanal 3 in Kontakt, insbesondere liegt die Trenneinrichtung 15 auf vorzugsweise säulenartigen Strukturen 17 o. dgl. im Kanal 3 in einem Einlaßbereich 18 des Kanals 3 auf. Die Strukturen 17 sind vorzugsweise mit keilartigen Ausnehmungen o. dgl. versehen (siehe Vergrößerung gemäß Fig. 2b), um das Blutplasma bzw. die Probenflüssigkeit 2 durch Kapillarkräfte von der Flachseite bzw. Abströmseite der Trenneinrichtung 15 zu der gegenüberliegenden Kanalfläche - hier zu der vom Träger 6 gebildeten Bodenfläche 8 des Kanals 3 - zu leiten und so eine vollständige Füllung zwischen Bodenfläche 8 und Abdeckung 7 bzw. des Einlaßbereichs 18 mit Probenflüssigkeit 2 zu bewirken. Jedoch können die Strukturen 17 auch andere geometrische Formen aufweisen und/oder miteinander direkt verbunden sein.

Die Strukturen 17 bilden eine Fülleinrichtung zum (vollständigen) Füllen des Kanals 3 zwischen Abdeckung 7 und Bodenfläche 8 mit Probenflüssigkeit 2. Die Fülleinrichtung kann jedoch auch in sonstiger Weise ausgebildet sein, wie später noch anhand der vierten Ausführungsform erläutert.

Anschließend wird die Probenflüssigkeit 2 - beim Darstellungsbeispiel nach Überwindung des ersten Grabens 11 - durch Kapillarkräfte weiter in den Kanal 3 gesaugt, wie durch die Hauptfüllrichtung F in Fig. 2 angedeutet.

Fig. 4 zeigt in einem schematischen Längsschnitt den bevorzugten Aufbau der vorschlagsgemäßen Vorrichtung 1 gemäß der ersten Ausführungsform, wobei zur Veranschaulichung ein zugeführter Tropfen Blut 19 angedeutet ist.

Die Trenneinrichtung 15 kann bedarfsweise eine Chemikalie, insbesondere eine Trockenchemikalie, enthalten, insbesondere um die beim Darstellungsbeispiel gewünschte Abtrennung von Blutplasma als Probenflüssigkeit 2 vom Blut 19 zu ermöglichen oder zu unterstützen und/oder bedarfsweise eine Lysierung von Zellen zu ermöglichen. Die Abtrennung bzw. Weiterleitung erfolgt insbesondere ausschließlich durch Kapillarkräfte.

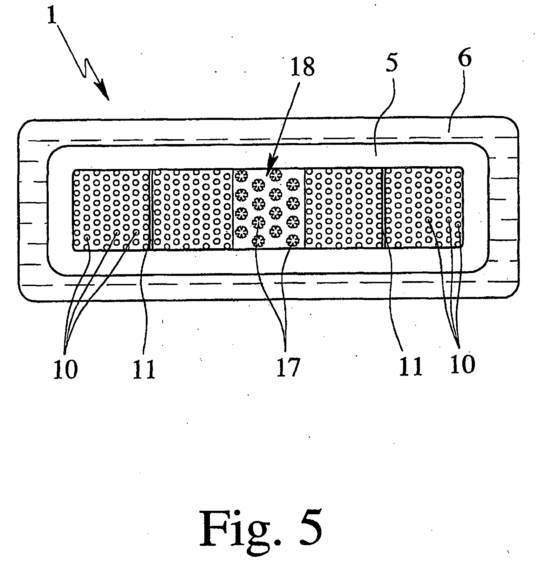

Vorzugsweise schließt sich an die Trenneinrichtung 15 nur ein einziger Kanal 3 zur Aufnahme oder Ableitung der Probenflüssigkeit 2 an. Hierbei ist der Kanal 3 vorzugsweise im Sinne einer Einzelkapillare ausgebildet bzw. zu verstehen. Bedarfsweise kann der Kanal 3 jedoch in unterschiedliche Richtungen oder zu unterschiedlichen Bereichen führen oder sich verzweigen, wie nachfolgend unter Bezugnahme auf die zweite Ausführungsform gemäß Fig. 5 erläutert.

Fig. 5 zeigt eine Draufsicht des Trägers 6 der Vorrichtung 1 gemäß der nicht beanspruchten zweiten Ausführungsform ohne Abdeckung 7. Hier erstreckt sich der Kanal 3 ausgehend von der Zuführeinrichtung 13 bzw. dem Zuführbereich 18 auf entgegengesetzte Seiten bzw. in entgegengesetzte Richtungen, beispielsweise um simultan unterschiedliche Untersuchungen, Tests o. dgl. durchzuführen. Beim Darstellungsbeispiel ergibt sich eine im wesentlichen längliche Anordnung. Jedoch sind beliebige andere Anordnungen möglich.

Bei der nicht beanspruchten zweiten Ausführungsform ist vorzugsweise wiederum die Ausnehmung 5 zur zumindest abschnittsweisen, seitenwandfreien Führung der Probenflüssigkeit 2 im Kanal 3 vorgesehen. Insbesondere umgibt die Ausnehmung 5 die gesamte Kanalkonfiguration vollständig, wobei der Kanal 3 vorzugsweise wiederum allseitig seitlich offen ausgebildet sein kann.

Bei der zweiten Ausführungsform gelten ansonsten die Erläuterungen zur ersten Ausführungsform.

Die Vorrichtung 1 gemäß der ersten und zweiten Ausführungsform weist jeweils eine Entlüftungseinrichtung auf, um die Probenflüssigkeit 2 im Einlaßbereich 18 quer zur Hauptfüllrichtung F des Kanals 3 und/oder quer zur Längserstreckung des Kanals 3, insbesondere in der Haupterstreckungsebene E des Kanals 3 zu entlüften. Vorhandene Luft oder sonstige Gase können seitlich aus dem Einlaßbereich 18 entweichen, wie beispielhaft durch Pfeile P in Fig. 3 angedeutet. Die Entlüftungseinrichtung gestattet insbesondere eine Entlüftung der Probenflüssigkeit 2 unmittelbar an der Abströmseite der Trenneinrichtung 15, also an der mit dem Einlaßbereich 18 des Kanals 3 in Kontakt stehenden Flachseite der Trenneinrichtung 15.

Die Entlüftungseinrichtung weist bei der ersten und zweiten Ausführungsform die sich seitlich an den Kanal 3 anschließende Ausnehmung 5 auf, um die gewünschte seitliche Entlüftung zu realisieren. Entsprechend genügt es für die vorschlagsgemäße Lösung, die Ausnehmung 5 ggf. nur entlang des Einlaßbereichs 18 und ggf. zusätzlich bei der ersten Ausführungsform entlang der Querseite des Einlaßbereichs 18 auszubilden. Die bei der ersten und zweiten Ausführungsform vorzugsweise vorgesehene Ausbildung der Ausnehmung 5 bzw. seitenwandlose Führung der Probenflüssigkeit 2 im Kanal 3 über den Einlaßbereich 18 hinaus entlang der Schmal- bzw. Längsseiten 4 des Kanals 3 ist hinsichtlich der Ausbildung einer gleichmäßigen Flüssigkeitsfront V, hinsichtlich des Verhinderns eines seitlichen Vorschießens der Probenflüssigkeit V und hinsichtlich der Optimierung der Entlüftung im weiteren Verlauf des Kanals 3 vorteilhaft, jedoch zur Realisierung der vorschlagsgemäßen Entlüftung im Einlaßbereich 18 nicht erforderlich.

Die vorschlagsgemäße Entlüftungseinrichtung bildet allgemein ausgedrückt mindestens einen Entlüftungspfad, der entlang der Abströmseite, insbesondere Flachseite, der Trenneinrichtung 15 und quer zur Abfluß- bzw. Hauptfüllrichtung F des Kanals 3 mit Probenflüssigkeit 2 verläuft, um eine optimale Füllung des Einlaßbereichs 18 mit Probenflüssigkeit 2 zu ermöglichen und insbesondere Lufteinschlüsse an der Abströmseite der Trenneinrichtung 15 zu verhindern.

Bei der ersten und zweiten Ausführungsform weist die vorschlagsgemäße Entlüftungseinrichtung vorzugsweise mindestens einen Entlüftungskanal 12 auf, der an die Ausnehmung 5 angeschlossen ist und die Ausnehmung 5 mit der Umgebung verbindet. Alternativ kann sich die Ausnehmung 5 auch direkt zur Umgebung hin öffnen.

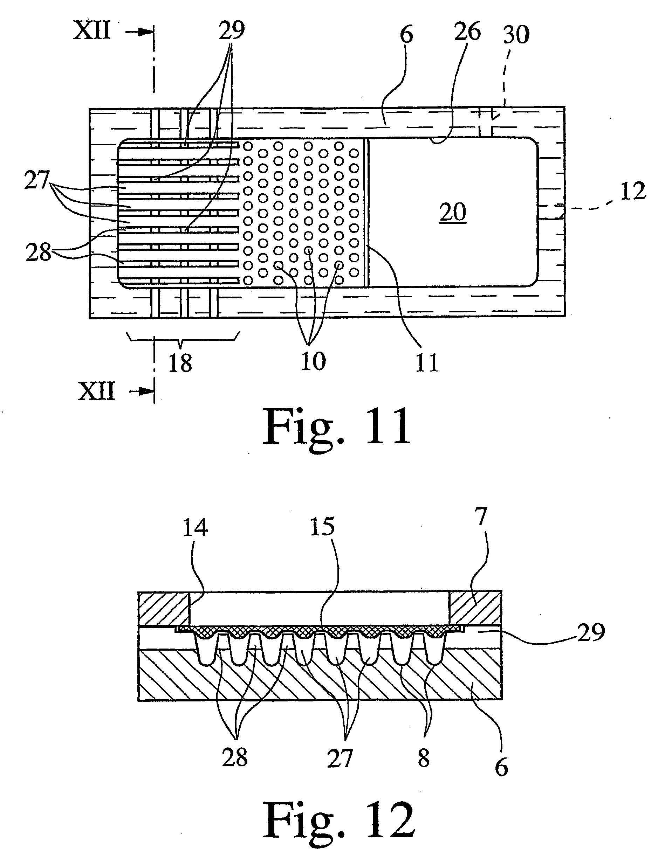

Die vorschlagsgemäße seitliche Entlüftung im Einlaßbereich 18 kann jedoch auch auf andere Weise realisiert werden, wie beispielhaft später anhand der siebten Ausführungsform unter Bezugnahme auf Fig. 11 und 12 erläutert.

Nachfolgend werden weitere Ausführungsformen der vorschlagsgemäßen Vorrichtung 1 erläutert, wobei primär nur wesentliche Unterschiede gegenüber den bisherigen Ausführungsformen hervorgehoben werden. Die bisherigen Erläuterungen gelten also ergänzend oder entsprechend.

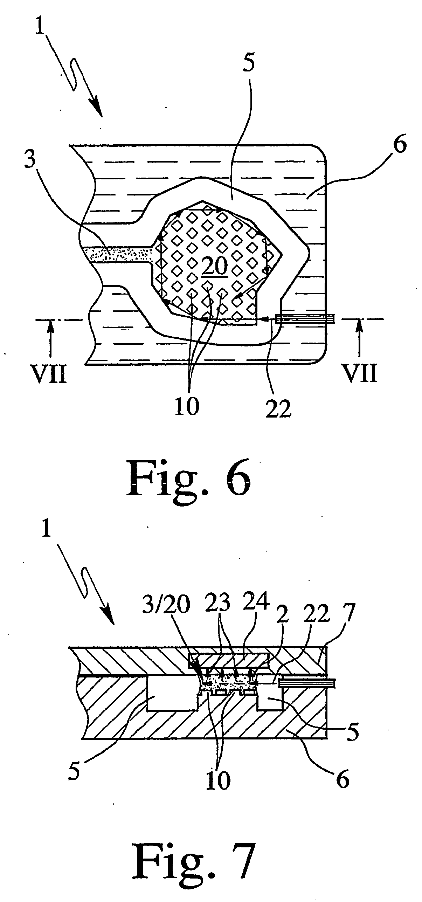

Fig. 6 und 7 zeigen eine nicht beanspruchte dritte Ausführungsform der vorschlagsgemäßen Vorrichtung 1, und zwar Fig. 6 eine Draufsicht des Trägers 6 ohne Abdeckung 7 und Fig. 7 eine Schnittansicht entlang Linie VII-VII von Fig. 6 bei vorhandener Abdeckung 7. Der Kanal 3 bildet hier eine Sammelkammer 20 für die Probenflüssigkeit 2. Die Sammelkammer 20 ist wiederum im wesentlichen flach ausgebildet und weist bedarfsweise die angedeuteten Erhöhungen 10 und/oder sonstige Leitelemente o. dgl. auf.

Die Vorrichtung 1 gemäß der dritten Ausführungsform weist eine Einrichtung 21, insbesondere einen Lichtleiter o. dgl., zur Einkopplung von Licht in die Probenflüssigkeit 2, insbesondere für Fluoreszenzmessungen, auf. Das Licht trifft im Bereich einer offenen Seite 4 des Kanals 3 auf die freie Oberfläche der Probenflüssigkeit 2 und tritt aufgrund einer entsprechend steilen, vorzugsweise im wesentlichen zur Flüssigkeitsoberfläche senkrechten Auftreffrichtung in die Probenflüssigkeit 2 ein, wie durch Pfeil 22 angedeutet. Für den Lichteintritt wird also die Grenzfläche Gas (Luft)/Probenflüssigkeit 2 genutzt. So kann vermieden werden, daß das Licht durch eine ansonsten vorhandene Seitenwand geleitet werden muß und dadurch in unerwünschter Weise gestreut wird oder Fluoreszenz hervorrufen kann.

Wie in Fig. 6 angedeutet, wird der eintretende Lichtstrahl 22 vorzugsweise mehrfach durch Totalreflexion an der Grenzfläche Probenflüssigkeit 2 / Gas (Luft) reflektiert. Dies wird dadurch erreicht, daß der Winkel zwischen Flächenlot und einfallendem Lichtstrahl jeweils größer als der Grenzwinkel der Totalreflexion ist. Die Grund- bzw. Bodenfläche der Sammelkammer 20, die durch die umlaufende Ausnehmung 5 begrenzt und definiert ist, ist entsprechend gewählt, um die gewünschte Strahlführung und Totalreflexion zu erreichen, beim Darstellungsbeispiel durch eine geeignete polygonale Konfiguration.

Das eingestrahlte Licht 22 dient der Fluoreszenzbestimmung bzw. Fluoreszenzspektroskopie. Die Probenflüssigkeit 2, insbesondere darin enthaltene Marker-Moleküle o. dgl., die beispielsweise als Chemikalie in Kanal 3 vorhanden sind und durch Probenflüssigkeit gelöst werden, werden durch eine bestimmte Wellenlänge angeregt. Dies führt zu Elektronenübergängen in den Molekülen, die nach einer gewissen Zeit unter Emission eines Photons in ihren Ursprungszustand zurückfallen. Die emittierte Strahlung ist in Fig. 8 durch Pfeile 23 angedeutet und mittels eines Detektors 24 detektierbar.

Um den Einfluß der Erhöhungen 10 oder sonstiger Leitelemente auf den einfallenden Lichtstrahl 22 auszuschließen, ist die Lichtstrahlebene oberhalb derartiger bzw. beabstandet zu derartigen Strukturen angeordnet. Des weiteren verläuft die Lichtstrahlebene zumindest im wesentlichen parallel zur Haupterstreckungsebene bzw. in der Haupterstreckungsebene E des Kanals 3 bzw. der Sammelkammer 20.

Die vorgesehene Lichteinstrahlung und Lichtführung gestatten eine weitgehend vollständige Anregung der Probenflüssigkeit 2 bzw. von darin enthaltenen Marker-Molekülen o. dgl. sowie gleichzeitig den Einsatz von Mikrostrukturen, wie den Erhebungen 10 oder sonstigen Leitelementen.

Die Erfassung der emittierten Lichtstrahlen 23 quer, insbesondere senkrecht, zur Einstrahlrichtung 22 ist hinsichtlich einer Entkopplung vom einfallenden Licht optimal.

Fig. 8 zeigt einen schematischen Längsschnitt einer beanspruchten vierten Ausführungsform der vorschlagsgemäßen Vorrichtung 1. Gegenüber der ersten Ausführungsform weist hier die Fülleinrichtung zur Füllung des Kanals 3 zwischen den beiden Flachseiten 8 und 9, insbesondere zur Ableitung des Blutplasmas bzw. der Probenflüssigkeit 2 von der Trenneinrichtung 15 bzw. von der Deckfläche 9 zu der gegenüberliegenden Bodenfläche 8, um einen räumlichen Meniskus zwischen den beiden Flächen bzw. Flachseiten 8 und 9 zu bilden, alternativ oder zusätzlich zu den Strukturen 17 eine Schräge bzw. Rampe 25 auf, die die Kanalhöhe H entsprechend verringert oder gegebenenfalls sogar zu Null werden läßt. Insbesondere kann die Trenneinrichtung 15 unmittelbar mit der Rampe 25 in Kontakt stehen bzw. darauf aufliegen. Die genannte Fülleinrichtung kann auch als Einrichtung zur Deckel- und Bodenbenetzung bereichnet bzw. verstanden werden.

Die schematische Schnittdarstellung gemäß Fig. 9a zeigt eine nicht beanspruchte fünfte Ausführungsform der vorschlagsgemäßen Vorrichtung 1. Hier ist die sich seitlich an den Kanal 3 anschließende Ausnehmung 5 durch die Probenflüssigkeit 2 füllbar und derart - insbesondere aufgrund einer entsprechenden Rundung ihrer Seitenwandung 26 und/oder durch Ausbildung von entsprechenden Leitelementen, wie Erhöhungen 10 oder dergleichen, wie in der ausschnittsweisen Vergrößerung gemäß Fig. 9b angedeutet - ausgebildet, daß die Füllgeschwindigkeit der Ausnehmung 5 in Hauptfüllrichtung F - bei der Darstellung gemäß Fig. 9a also senkrecht zur Zeichenebene - nicht die Füllgeschwindigkeit des Kanals 3 überschreitet, um das unerwünschte seitliche Vorschießen der Flüssigkeitsfront V zu verhindern. Anzumerken ist hierbei, daß beim Darstellungsbeispiel die Höhe H der Ausnehmung 5 etwa nur der Höhe H des Kanals 3 entspricht. Vorzugsweise ist diese jedoch größer.

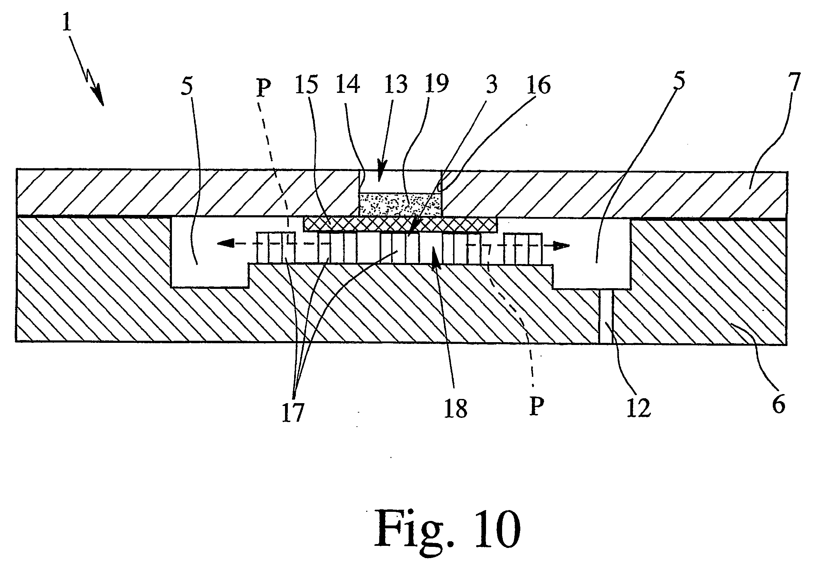

Fig. 10 zeigt eine nicht beanspruchte sechste Ausführungsform der vorschlagsgemäßen Vorrichtung 1 in einer schematischen Schnittdarstellung im Einführbereich 18 quer zur Hauptströmungs- bzw. Hauptfüllrichtung F des Kanals 3, also entsprechend Fig. 3.

Bei der sechsten Ausführungsform weist die Trenneinrichtung 15 ein flaches Filterelement, insbesondere eine dünne Membran auf, die auf der dem Kanal 3 bzw. dessen Einlaßbereich 18 zugewandten Seite 9 der Abdeckung 7 angeordnet ist. Die Membran überdeckt die Durchbrechung 14 vorzugsweise vollständig und ist insbesondere durch Schweißen, Kleben oder dgl. dicht mit der Abdeckung 7 verbunden. Die Membran steht vorzugsweise unmittelbar mit der darunter angeordneten Fülleinrichtung, insbesondere deren Strukturen 17, in Kontakt, um - wie bereits erläutert - eine optimale Füllung des Einlaßbereichs 18 mit Probenflüssigkeit 2 zu ermöglichen. Jedoch kann die Membran ggf. auch von der darunter liegenden Fülleinrichtung beabstandet sein.

Fig. 10 veranschaulicht insbesondere nochmals den Vorgang bei der Bluttrennung. Blut 19 wird von der Zuführeinrichtung 13 aufgenommen und bedeckt die Trenneinrichtung 15 vorzugsweise vollständig, um die gesamte zur Verfügung stehende Fläche auszunutzen und dadurch eine möglichst schnelle Bluttrennung zu ermöglichen.

Die Membran ist so ausgebildet, daß Blutzellen, insbesondere rote Blutkörperchen, zurückgehalten werden. Vorzugsweise nimmt die Kapillarität in der Membran zur Abströmseite hin zu, um Blutplasma oder sonstige Blutbestandteile als Probenflüssigkeit 2 abzutrennen und auf der Abströmseite zur Verfügung zu stellen.

Der Einlaßbereich 18 nimmt dann - auch bei geringerer Kapillarität der Fülleinrichtung als die Membran, wenn Probenflüssigkeit 2 von der Zuführseite aus dem Blut 19 nachströmt - die abgetrennte Probenflüssigkeit 2 auf. Der Einlaßbereich 18 schließt sich unmittelbar und vollflächig an die Abströmseite bzw. Flachseite der Trenneinrichtung 15 bzw. Membran an und leitet die Probenflüssigkeit 2 - zumindest nach vollständiger Füllung mit Probenflüssigkeit 2 - zumindest im wesentlichen parallel zu der Flachseite in Hauptfüllrichtung F ab.

Ein nicht beanspruchtes Verfahren zeichnet sich dadurch aus, daß die Probenflüssigkeit 2 zumindest im Einsatzbereich 18 quer zur Hauptfüllrichtung F entlüftet wird. Insbesondere erfolgt die Entlüftung in der Haupterstreckungsebene E des Kanals 3 und/oder parallel zur Flachseite der Trenneinrichtung 15. Durch die vorschlagsgemäße Entlüftung wird ein vollständiges Füllen des Einlaßbereichs 18 ermöglicht und insbesondere werden unerwünschte Lufteinschlüsse und damit potentielle Blockierungen der Trenneinrichtung 15 vermieden, wie bereits erläutert.

Fig. 11 und 12 zeigen eine beanspruchte siebte Ausführungsform der vorschlagsgemäßen Vorrichtung 1 ohne Probenflüssigkeit 2. Fig. 11 zeigt den Träger 6 ohne Abdeckung 7. Fig. 12 zeigt eine Schnittansicht entlang Linie XII-XII von Fig. 11 bei vorhandener Abdeckung 7.

Im Gegensatz zu den bisherigen Ausführungsformen weist der Kanal 3 bei der siebten Ausführungsform - zumindest im Einlaßbereich 18 - mehrere Einzelkanäle 27 auf, die durch Trennwände 28 voneinander getrennt sind. Die Einzelkanäle 27 verlaufen vorzugsweise parallel zueinander in Hauptfüllrichtung F des Kanals 3.

Die Schnittdarstellung gemäß Fig. 12 veranschaulicht, daß die Trenneinrichtung 15, insbesondere in Form eines flachen Filterelements oder einer Membran, unmittelbar auf den Trennwänden 28 aufliegt und ggf. wellenartig in die Emzelkanäle 27 gewölbt ist bzw. die Trennwände 28 in die Trenneinrichtung 15 eingedrückt sein können. Dies erleichtert das Füllen der Einzelkanäle 27 mit Probenflüssigkeit 2.

Die Einzelkanäle 27 verjüngen sich im Querschnitt zum Boden 8, also zu der der Trenneinrichtung 15 abgewandten Seite, hin, insbesondere im Querschnitt im wesentlichen V-förmig. Dies ist des weiteren einem vollständigen Füllen mit Probenflüssigkeit 2 - insbesondere bei entsprechender Dimensionierung der Einzelkanäle 27 - zuträglich. Die genannte Querschnittsverjüngung kann auch unabhängig von den Einzelkanälen 27 - also im Einlaßbereich 18 und/oder in sonstigen Kanalbereichen - beim Kanal 3 verwirklicht sein. Die Querschnittsverjüngung kann auch als Fülleinrichtung bzw. Mittel zum - schnellen bzw. vollständigen - Füllen mit Probenflüssigkeit 2 angesehen werden und unabhängig von der vorliegenden Ausführungsform und unabhängig von der vorschlagsgemäßen Entlüftung realisiert werden.

Bei der siebten Ausführungsform ist die Probenflüssigkeit 2 vorzugsweise nicht seitenwandfrei im Kanal 3 gerührt. Vielmehr ist der Kanal 3 zumindest im wesentlichen seitlich begrenzt, beim Darstellungsbeispiel von einer umlaufenden Seitenwandung 26 des Trägers 6, wie in Fig. 11 dargestellt. Bedarfsweise kann die Seitenwandung 26 entsprechend der fünften Ausführungsform mit einer Einrichtung zum Verhindern des seitlichen Vorschießens der Probenflüssigkeit 2 im Kanal 3 - insbesondere seitlichen Leitelementen, Erhöhungen 10 oder dgl., wie insbesondere in Fig. 10b angedeutet - versehen und/oder gerundet ausgebildet sein.

Zur vorschlagsgemäßen Entlüftung weist die Entlüftungseinrichtung bei der siebten Ausführungsform mindestens einen Querkanal 29 im Einlaßbereich 18 auf, der sich quer zur Hauptfüllrichtung F des Kanals 3 und insbesondere parallel zur Abströmseite bzw. Flachseite der Trenneinrichtung 15 erstreckt. Insbesondere ist der Querkanal 29 über seine gesamte Länge zur Trenneinrichtung 15 hin offen, um eine optimale Entlüftung zu ermöglichen.

Beim Darstellungsbeispiel weist die Entlüftungseinrichtung mehrere Querkanäle 29 auf, die vorzugsweise parallel zueinander verlaufen, um eine optimale Entlüftung des Einlaßbereiches 18 - auch bei nicht seitenwandloser Führung der Probenflüssigkeit 2 im Einlaßbereich 18 - zu ermöglichen. Die Querkanäle 29 können dann über nicht dargestellte Sarmmelkammern oder über separate Entlüftungskanäle 12 mit der Umgebung in Verbindung stehen, um die gewünschte Entlüftung zu ermöglichen.

Beim Darstellungsbeispiel schneiden die Querkanäle 29 die Zwischenwände 28 bedarfsweise nur in einem oberen Bereich, also nicht über die gesamte Höhe H des Kanals 3. Dies ist jedoch bei Bedarf auch möglich.

Die Querschnitte oder außenseitigen Öffnungen der Querkanäle 23 sind so ausgelegt, daß Probenflüssigkeit 2 durch die Querkanäle 29 nicht entweichen kann. Alternativ oder zusätzlich können auch nicht dargestellte Kapillarstops, hydrophob oder hydrophil beschichtete Bereiche, nur gasdurchlässig Materialien oder dgl. vorgesehen sein, um ein unerwünschtes Entweichen von Probenflüssigkeit 2 durch die Querkanäle 29 zu verhindern. Alternativ kann auch ein gewisses Lecken von Probenflüssigkeit 2 durch die Querkanäle 29 in Kauf genommen werden, um ein schnelles Füllen des Kanals 3 im Einlaßbereich 18 mit Probenflüssigkeit 2 und/oder eine optimale Entlüftung zu erreichen.

Die vorschlagsgemäße Entlüftung kann nicht nur bei den dargestellten, sondern auch bei sonstigen, insbesondere der Bluttrennung oder anderen insbesondere biologischen oder medizinischen Untersuchungen dienenden mikrofluidischen Vorrichtungen zur Abtrennung von Probenflüssigkeit 2 eingesetzt werden.

Einzelne Merkmale der verschiedenen Ausführungsformen können beliebig miteinander kombiniert werden.

Die Flachseiten 8, 9 des Kanals 3 können bedarfsweise auch glatt ausgebildet sein, insbesondere also keine Texturierungen, Mikrostrukturierungen, Leitelemente, Erhebungen 10 oder dgl. aufweisen. Beispielsweise ist der Sammelbereich 20 bei der siebten Ausführungsform glatt ausgebildet.

Die vorschlagsgemäßen Vorrichtungen 1 sind für verschiedenste Tests, Untersuchungen o. dgl. geeignet. Insbesondere gestatten sie immunologische oder biochemische Test, beispielsweise von Blut 19, Blutplasma o. dgl.

Gemäß einer Ausführungsvariante kann der Kanal 3 mehrere Untersuchungsbereiche bzw. Sammelbereiche 20 bilden, die nacheinander mit der Probenflüssigkeit 2 füllbar sind. So ist es beispielsweise möglich, verschiedene Untersuchungen nacheinander durchzuführen und/oder die Probenflüssigkeit 2 nacheinander verschiedenen Reagenzien, insbesondere Trockenchemikalien, die nacheinander gelöst werden, auszusetzen.

Gemäß einer anderen Ausführungsvariante kann sich an einen ersten Untersuchungs- oder Sammelbereich 20 ein zweiter Untersuchungs- oder Sammelbereich 20 anschließen, wobei der zweite Bereich vorzugsweise eine wesentlich höhere Kapillarität, beispielsweise durch ein eingesetztes Vlies o. dgl., aufweist. So kann die Probenflüssigkeit 2 nach Füllen des ersten Bereichs und insbesondere nach Lösen einer bedarfsweise dort vorhandenen Trockenchemikalie anschließend in den zweiten Bereich gesaugt bzw. geleitet werden, wobei die Trockenchemikalie aus dem ersten Bereich ausgewaschen und so beispielsweise eine weitere Untersuchung im ersten und/oder zweiten Bereich ermöglicht wird.

Vorzugsweise nimmt die Kapillarität des Kanals 3 ausgehend vom Einlaßbereich 18 zum anderen Ende hin zu. Dies kann dazu führen, daß sich der Kanal 3 vom Einlaßbereich 18 her leert, wenn nicht ausreichend Probenflüssigkeit 2 nachströmt. Alternativ kann die Kapillarität im Kanal 3 auch gleichmäßig oder ausgehend vom Einlaßbereich 18 zum anderen Ende hin abnehmend ausgebildet sein, um den Effekt des möglichen Leerens des Kanals 3 ausgehend vom Einlaßbereich 18 zu vermeiden.

Gemäß einer weiteren Ausführungsvariante sind eine erste Chemikalie, insbesondere eine Trockenchemikalie, vorzugsweise in der Zuführeinrichtung 13 oder Trenneinrichtung 15, und mindestens eine zweite Chemikalie, insbesondere eine Trockenchemikalie, vorzugsweise im Kanal 3 bzw. Sammelbereich 20, vorgesehen. Dies gestattet eine effektive Manipulation bzw. Beeinflussung der Probenflüssigkeit 2, des Bluts 19 o. dgl.

Vorzugsweise ist zur Untersuchung von Blutplasma die erste Chemikalie derart ausgebildet, daß diese eine Gerinnung des Bluts 19 verhindert bzw. verzögert. Hierzu kann beispielsweise zur Herstellung von EDTA-Blut als erste Chemikalie EDTA (Ethylene Diamine Tetraacetic Acid (Äthylendiamintetraessigsäure)) verwendet werden. Dabei bindet das EDTA das Calcium des Bluts, das als Faktor IV für die Blutgerinnung erforderlich ist.

Anschließend dient die zweite Chemikalie, vorzugsweise ein Chemikaliengemisch, einer Untersuchung bzw. einer Bestimmung eines oder mehrerer Parameter im Blutplasma, wie Glukose, Ketone oder Laktat.

Vorzugsweise ist zur Untersuchung mindestens eines intrazellulären Parameters, wie des Hämoglobin-Werts oder des Calcium-Werts in Blut 19, die erste Chemikalie derart ausgebildet, daß sie Zellen, wie Blutzellen, lysiert und das Calcium o. dgl. freisetzt. Hierzu wird beispielsweise Lysinpuffer eingesetzt.

Anschließend dient die zweite Chemikalie, vorzugsweise ein Chemiekaliengemisch, einer Untersuchung bzw. Bestimmung des Parameters, insbesondere des Calcium-Gehalts. Ein Bestandteil des Gemisches, vorzugsweise der Chelatbildner 8-hydroxychinolin, wird dazu eingesetzt, die Reaktion störende Magnesiumionen aus der Reaktion zu entfernen. Ein anderer Komplexbildner, vorzugsweise o-Kresolphthalein, bildet mit Calcium unter alkalischen Bedingungen einen farbigen Komplex.

Die Extinktion des Farbkomplexes ist bei einer Wellenlänge von 570 nm proportional zur Calcium-Konzentration. Sie wird direkt im Kanal 3 bzw. Sammelbereich 20 oder ggf. nach Entnahme bestimmt. Jedoch sind auch andere Bestimmungen oder Verfahrensweisen möglich. Insbesondere kann die Extinktion auch bei anderen Wellenlängen und/oder zur Bestimmung anderer Komplexe, Parameter o. dgl. eingesetzt werden. Entsprechendes gilt für sonstige, vorzugsweise optische Bestimmungsverfahren, wie Fluoreszenzmessungen o. dgl.

Gemäß noch einer weiteren Ausführungsvariante ist in der Abdeckung 7 und/oder im Träger 6 eine in Fig. 8 und 11 angedeutete Entnahmeöffnung 30 vorgesehen, um eine Entnahme der Probenflüssigkeit 2, insbesondere von abgetrenntem Blutplasma o. dgl., zu ermöglichen. Die Entnahmeöffnung 30 steht vorzugsweise mit einem zumindest relativ großvolumigen Speicherbereich 20 o. dgl. des Kanals 3 in Verbindung, um ein gewünschtes bzw. ausreichendes Entnahmevolumen bereitstellen zu können.

## Patentansprüche

1. Vorrichtung (1) zur Aufnahme von Blut (19) und Abtrennung von Blutbestandteilen, wie Blutplasma, als Probenflüssigkeit (2), mit einer Zuführeinrichtung (13) zur Aufnahme des Bluts (19), einer Trenneinrichtung (15) zur Abtrennung von Blutbestandteilen als Probenflüssigkeit (2) und einem die Probenflüssigkeit (2), vorzugsweise ausschließlich, durch Kapillarkräfte aufnehmenden Kanal (3), wobei sich ein Einlassbereich (18) des Kanals (3) an die Trenneinrichtung (15) zur Aufnahme der abgetrennten Probenflüssigkeit (2) anschließt, wobei die Vorrichtung (1) eine Entlüftungseinrichtung aufweist, um die Probenflüssigkeit (2) im Einlassbereich (18) quer zur Hauptfüllrichtung (F) und/oder Längserstreckung des Kanals (3) zu entlüften, und wobei die Vorrichtung (1) eine Fülleinrichtung zum Füllen des Kanals (3) mit Probenflüssigkeit (2) zwischen gegenüberliegenden, den Kanal (3) begrenzenden Flächen oder Flachseiten (8, 9) im Einlassbereich (18) aufweist.
**dadurch gekennzeichnet,**
**dass** die Fühleinrichtung eine die Höhe (H) des Kanals (3) im Einlassbereich (18) verringemde Rampe (25) aufweist, und/oder dass sich der Querschnitt des Kanals (3) im Einlassbereich (18) ausgehend von der Trenneinrichtung (15) zum Boden (8) hin verjüngt.

2. Vorrichtung nach Anspruch 1, **dadurch gekennzeichnet, dass** die Entlüftungseinrichtung zur Entlüftung der Probenflüssigkeit (2) unmittelbar an der Abströmseite der Trenneinrichtung (15) ausgebildet ist, und/oder dass die Entlüftungseinrichtung zur Entlüftung der Probenflüssigkeit (2) im Einlassbereich (18) in der Haupterstreckungsebene (E) des Kanals (3) und/oder entlang einer Flachseite der Trenneinrichtung (15) und/oder quer zur Strömungsrichtung der Probenflüssigkeit (2) durch die Trenneinrichtung (15) ausgebildet ist, und/oder dass die Entlüftungseinrichtung mindestens einen quer zur Hauptfüllrichtung (F) des Kanals (3) mit Probenflüssigkeit (2) verlaufenden Querkanal (30) im Einlassbereich (18) aufweist.

3. Vorrichtung nach einem der voranstehenden Ansprüche, **dadurch gekennzeichnet, dass** die Entlüftungseinrichtung eine sich seitlich an den Kanal (3) anschließende Ausnehmung (5) aufweist.

4. Vorrichtung nach Anspruch 3, **dadurch gekennzeichnet, dass** sich die Ausnehmung (5) zur Bildung eines Flüssigkeitsstopps scharfkantig an den Kanal (3) anschließt, und/oder dass die Ausnehmung (5) längs einer Hauptfüllrichtung (F) des Kanals (3) mit der Probenflüssigkeit (2) und/oder entlang der vorzugsweise gegenüberliegenden offenen Schmal- und/oder Längsseiten (4) des Kanals (3) verläuft, und/oder dass die Ausnehmung (5) umlaufend ausgebildet ist, insbesondere den Kanal (3) allseitig seitlich umgibt oder begrenzt.

5. Vorrichtung nach Anspruch 3 oder 4, **dadurch gekennzeichnet, dass** die Ausnehmung (5) im Querschnitt zumindest im wesentlichen rechteckig ausgebildet ist, und/oder dass die Ausnehmung (5) einseitig oder beidseitig bezüglich einer seitlichen Projektion des Kanals (3) ausgenommen ist, und/oder dass die Vorrichtung (1) einen Träger (6) und eine Abdeckung (7) aufweist, zwischen oder von denen der Kanal (3) und die Ausnehmung (5) gebildet sind, und/oder dass die Entlüftungseinrichtung mindestens einen an die Ausnehmung (5) angeschlossenen Entlüftungskanal (12) aufweist.

6. Vorrichtung nach einem der voranstehenden Ansprüche, **dadurch gekennzeichnet, dass** die Probenflüssigkeit (2) vom Einlassbereich (18) senkrecht zur Haupterstreckungsebene (E) des Kanals (3), insbesondere in Gebrauchslage vertikal, aufnehmbar ist, und/oder dass die Trenneinrichtung (15) flächig ausgebildet ist, wobei sich der Einlassbereich (18) unmittelbar an eine Flachseite der Trenneinrichtung (15) anschließt und die Hauptfüllrichtung (F) des Kanals (3) insbesondere parallel zur Flachseite verläuft, und/oder dass die Trenneinrichtung (15) einen Filter oder eine Membran zur Abtrennung von Blutplasma als Probenflüssigkeit (2) aufweist.

7. Vorrichtung nach einem der voranstehenden Ansprüche, **dadurch gekennzeichnet, dass** die Fülleinrichtung mindestens eine vorzugsweise steg-, stufen-, höcker- oder säulenartige Struktur (17) aufweist, die von einer Fläche oder Flachseite (8, 9) abragt.

8. Vorrichtung nach einem der voranstehenden Ansprüche, **dadurch gekennzeichnet, dass** sich der Querschnitt des Kanals (3) ausgehend von der Trenneinrichtung (15) zum Boden (8) hin, im wesentlichen V-förmig verjüngt.

9. Vorrichtung nach einem der voranstehenden Ansprüche, **dadurch gekennzeichnet, dass** der Kanal (3) zumindest im Einlassbereich (18) mehrere Einzelkanale (27) aufweist und/oder vorzugsweise nur von zwei gegenüberliegenden, insbesondere im wesentlichen ebenen, Flächen oder Flachseiten (8, 9) begrenzt oder gebildet ist.

10. Vorrichtung nach einem der voranstehenden Ansprüche, **dadurch gekennzeichnet, dass** der Kanal (3) an gegenüberliegenden oder allen Schmal- bzw. Längsseiten (4), insbesondere umlaufend seitlich, offen ausgebildet und die Probenflüssigkeit (2) seitenwandfrei im Kanal (3) führbar ist, und/oder dass der Kanal (3) einen im wesentlichen rechteckigen und/oder flachen Querschnitt, insbesondere quer zur Hauptfüllrichtung (F) mit Probenflüssigkeit (2) oder quer zur Längserstreckung des Kanals (3), aufweist.

11. Vorrichtung nach einem der voranstehenden Ansprüche, **dadurch gekennzeichnet, dass** die Höhe (H) des Kanals (3), insbesondere mindestens um den Faktor 5, kleiner als die Breite (B) des Kanals (3) ist und/oder dass das Aufnahmevolumen des Kanals (3) weniger als 1 ml, insbesondere weniger als 100 µl, besonders bevorzugt maximal 10 µl, beträgt, und/oder dass der Kanal (3) mindestens einen Speicher für die Probenflüssigkeit (2) bildet, und/oder dass der Kanal (3) eine Chemikalie, insbesondere eine Trockenchemikalie, enthält.

12. Vorrichtung nach einem der voranstehenden Ansprüche, **dadurch gekennzeichnet, dass** der Kanal (3) mindestens ein Leitelement zur Beeinflussung, insbesondere Vergleichmäßigung, des Füllens mit der Probenflüssigkeit (2) aufweist, und/oder dass der Kanal (3) vorzugsweise regelmäßig verteilte Erhöhungen (10) als Leitelemente aufweist, die insbesondere in Reihen quer oder längs zur Hauptfüllrichtung (F) angeordnet sind.

13. Vorrichtung nach Anspruch 12, **dadurch gekennzeichnet, dass** die Erhöhungen (10) höckerartig oder säulenartig, insbesondere mit runder oder polygonaler Grundfläche, ausgebildet sind, und/oder dass die Flächendichte, der Abstand und/oder die Größe der Leitelemente variiert, insbesondere in Abhängigkeit von der jeweiligen Entfernung zu einem Einlass für die Probenflüssigkeit (2) in den Kanal (3).

14. Vorrichtung nach Anspruch 12 oder 13, **dadurch gekennzeichnet, dass** der Kanal (3) mindestens einen Graben (11) als Leitelement aufweist, der insbesondere quer oder längs zur Hauptfüllrichtung (F) des Kanals (3) verläuft und vorzugsweise einen nur temporären Flüssigkeitsstopp zur Vergleichmäßigung der Flüssigkeitsfront (V) der Probenflüssigkeit (2) bildet, insbesondere wobei der Graben (11) einen zumindest im wesentlichen rechteckigen oder halbrunden Querschnitt aufweist und/oder seine Tiefe geringer als die Tiefe der Ausnehmung (5) ist.

15. Vorrichtung nach einem der voranstehenden Ansprüche, **dadurch gekennzeichnet, dass** die Zuführeinrichtung (13) und/oder Trenneinrichtung (15) eine erste Chemikalie, insbesondere ein Mittel zur Lysierung von Zellen, vorzugsweise von Blutzellen, aufweist, insbesondere wobei die erste Chemikalie als Trockenchemikalie vorliegt und von zugeführter Flüssigkeit, wie der Probenflüssigkeit (2) und/oder Blut (19), lösbar ist.

16. Vorrichtung nach einem der voranstehenden Ansprüche, **dadurch gekennzeichnet, dass** der Kanal (3), insbesondere dessen Sammelbereich (20), mit mindestens einer zweiten Chemikalie versehen ist, insbesondere wobei die zweite Chemikalie als Trockenchemikalie vorliegt und von der Probenflüssigkeit (2), insbesondere Blutplasma und/oder intrazellulärer Flüssigkeit, lösbar ist, und/oder insbesondere wobei die zweite Chemikalie eine Bestimmung des Ca-Gehalts ermöglicht und/oder einen Komplexbildner, insbesondere o-Kresolphthalein, zur Bildung eines farbigen Komplexes enthält

17. Vorrichtung nach einem der voranstehenden Ansprüche, **dadurch gekennzeichnet, dass** sich an die Trenneinrichtung (15) nur ein einziger Kanal (3) zur Aufnahme und/oder Ableitung der Probenflüssigkeit (2) anschließt.

18. Vorrichtung nach einem der voranstehenden Ansprüche, **dadurch gekennzeichnet, dass** die Vorrichtung (1) einen vorzugsweise an einer Flachseite (9) des Kanals (3) angeordneten Detektor (24) zur Streulicht- oder Fluoreszenzmessung aufweist, und/oder dass die Vorrichtung (1) eine Entnahmeeinrichtung zur Entnahme von Probenflüssigkeit (2) aufweist.

19. Verwendung einer Vorrichtung (1) nach einem der Ansprüche 1 bis 18 zur Bestimmung mindestens eines Bestandteils in Blut (19) bzw. zur Analyse von Blutplasma, insbesondere von Proteinen oder Elektrolyten, als Probenflüssigkeit (2), wobei die Probenflüssigkeit (2), vorzugsweise ausschließlich, durch Kapillarkräfte des Kanals (3) gefördert und/oder gefiltert wird und wobei mittels einer Chemikalie ein oder mehrere Bestandteile und/oder Parameter, vorzugsweise unmittelbar im Kanal (3) oder einem sich anschließenden Sammelbereich (20), bestimmt wird bzw. werden.

20. Verwendung nach Anspruch 19, **dadurch gekennzeichnet, dass** in der Probenflüssigkeit (2), insbesondere im Blutplasma, der Gehalt an Glukose, Ketonen oder Laktat, mittels der Chemikalie bestimmt wird, und/oder dass die Blutgerinnung mittels einer anderen Chemikalie verhindert wird, insbesondere wobei im Blut (19) vorhandenes Fibrinogen mittels der anderen Chemikalie ausgefällt und gefiltert bzw. separiert wird und in der entstehenden Probenflüssigkeit (2) der Parameter bestimmt wird.

21. Verwendung nach Anspruch 19 oder 20, **dadurch gekennzeichnet, dass** der Parameter oder Blutbestandteil optisch oder elektrochemisch in der Probenflüssigkeit (2), insbesondere unmittelbar im Kanal (3) oder in einem davon gebildeten Sammelbereich (20), bestimmt wird.

## Claims

1. Device (1) for collecting blood (19) and separating blood components, such as blood plasma, as sample liquid (2), having a feed device (13) for collecting the blood (19), a separator (15) for separating blood components as sample liquid (2), and a channel (3) receiving the sample liquid (2) preferably exclusively by capillary forces, wherein subsequent to the separator (15) there is an inlet region (18) of the channel (3) for receiving the separated sample liquid (2), wherein the device (1) comprises a venting device for venting the sample liquid (2) in the inlet region (18) at right-angles to the main filling direction (F) and/or longitudinal extension of the channel (3), and wherein the device (1) comprises a filling device for filling the channel (3) with the sample liquid (2) between opposing surfaces or flat sides (8, 9) in the inlet region (18) which delimit the channel (3),
**characterized in**
**that** the filling device comprises a ramp (25) which reduces the height (H) of the channel (3) in the inlet region (18), and/or that the cross-section of the channel (3) in the inlet region (18) tapers from the separator (15) towards the base (8).

2. Device according to claim 1, **characterized in that** the venting device is constructed to vent the sample liquid (2) immediately at the outflow side of the separator (15), and/or that the venting device for venting the sample liquid (2) in the inlet region (18) is formed in the main extension plane (E) of the channel (3) and/or along a flat side of the separator (15) and/or at right-angles to the direction of flow of the sample liquid (2) through the separator (15), and/or that the venting device comprises at least one transvers channel (3) in the inlet region (18), extending at right angles to the main filling direction (F) of the channel (3) with sample liquid (2).

3. Device according to one of the preceding claims, **characterized in that** the venting device has a recess (5) laterally adjacent to the channel (3).

4. Device according to claim 3, **characterized in that** the recess (5) joins the channel (3) in a sharp-edged manner to form a liquid stop, and/or that the recess (5) runs along the main filling direction (F) of the channel (3) with the sample liquid (2) and/or along the preferably opposite open narrow and/or longitudinal sides (4) of the channel (3), and/or that the recess (5) is of circumferential construction, in particular surrounds or delimits the channel (3) literally all around.

5. Device according to claim 3 or 4, **characterized in that** the recess (5) is at least substantially rectangular in cross-section, and/or that the recess (5) is formed on one or both sides with respect to a lateral projection of the channel (3), and/or that the device (1) comprises a carrier (6) and cover (7) between which or by which the channel (3) and recess (5) are formed, and/or that the venting device comprises at least one venting channel (12) connected to the recess (5).

6. Device according to one of the preceding claims, **characterized in that** a sample liquid (2) can be received by the inlet region (18) perpendicular to the main extension plane (E) of the channel (3), particularly vertically in the position of use, and/or that the separator (15) is of planar construction, the inlet region (18) being immediately adjacent to a flat side of the separator (15) and the main filling direction (F) of the channel (3) extending, in particular parallel, to the flat side, and/or that the separator (15) comprises a filter or a membrane for separating blood plasma as a sample liquid (2).

7. Device according to one of the preceding claims, **characterized in that** the filling device comprises at least one structure (17) preferably in the form of a bar, step, bump or column, projecting from a surface or flat side (8, 9).

8. Device according to one of the preceding claims, **characterized in that** the cross-section of the channel (3) tapers from the separator (15) towards the base (8) substantially in V-shape.

9. Device according to one of the preceding claims, **characterized in that** the channel (3) comprises a plurality of individual channels (27) at least in the inlet region (18) and/or is preferably limited or formed only by two opposing surfaces or flat sides (8,9) which are, in particular, substantially planar.

10. Device according to one of the preceding claims, **characterized in that** the channel (3) is of open construction on opposing or all narrow and/or longitudinal sides (4), particularly in a laterally encircling manner, and the sample liquid (2) can be guided along the channel (3) without sidewalls, and/or that the channel (3) comprises a substantially rectangular and/or flat cross-section, particularly at right-angles to the main filling direction (F) with sample liquid (2) or at right-angles to the longitudinal dimension of the channel (3).

11. Device according to one of the preceding claims, **characterized in that** the height (H) of the channel (3) is less than the width (B) of the channel (3), particularly by at least a factor of 5, and/or that the capacity of the channel (3) is less than 1 ml, particularly less than 100 µl, most preferably not more than 10 µl, and/or that the channel (3) forms at least one reservoir for the sample liquid (2) and/or that the channel (3) contains a chemical, particularly a dry chemical.

12. Device according to one of the preceding claims, **characterized in that** the channel (3) comprises at least one guide element for influencing, particularly evening out, the filling with the sample liquid (2), and/or that the channel (3) comprises preferably regularly distributed elevations (10) as guide elements, arranged particularly in rows at right-angles to or longitudinally with respect to the main filling direction (F).

13. Device according to claim 12, **characterized in that** the elevations (10) are constructed in the manner of bumps or columns, particularly with a round or polygonal base area, and/or that the surface density, the spacing and/or the size of the guide elements varies, particularly as a function of the respective distance from an inlet for the sample liquid (2) into the channel (3).

14. Device according to claim 12 or 13, **characterized in that** the channel (3) comprises at least one trench (11) as a guide element, extending in particular transversally or longitudinally with respect to the main filling direction (F) of the channel (3) and forms preferably a only temporary liquid stop for evening out the liquid front (V) of the sample liquid (2), in particular wherein the trench (11) comprises an at least substantially rectangular or semicircular cross-section and/or its depth is less than the depth of the recess (5).

15. Device according to one of the preceding claims, **characterized in that** the feed device (13) and/or separator (15) comprises a first chemical, particularly an agent for lysing cells, preferably blood cells, particularly wherein the first chemical is in the form of a dry chemical and can be dissolved by liquid supplied, such as the sample liquid (2) and/or blood (19).

16. Device according to one of the preceding claims, **characterized in that** the channel (3), in particular its collecting region (20) is provided with at least one second chemical, preferably wherein the second chemical is in the form of a dry chemical and can be dissolved by the sample liquid (2), particularly blood plasma and/or intracellular liquid, and/or particularly wherein the second chemical allows the Ca content to be determined and/or contains a complexing agent, particularly o-Cresolphthalein, to form a coloured complex.

17. Device according to one of the preceding claims, **characterized in that** subsequent to the separator (15) there is a single channel (3) for receiving and/or discharging the sample liquid (2).

18. Device according to one of the preceding claims, **characterized in that** the device (1) comprises a detector (24) preferably mounted on a flat side (9) of the channel (3), for measuring scattered light or fluorescence, and/or that the device (1) comprises a sampling device for sampling sample liquid (2).

19. Use of a device (1) according to one of the claims 1 to 18 for determining at least one component in blood (19) and/or for analyzing blood plasma, particularly proteins or electrolytes, as sample liquid (2), wherein the sample liquid (2) is conveyed and/or filtered, preferably exclusively, by capillary forces of the channel (3), and wherein one or more components and/or parameters is or are determined by means of a chemical, preferably directly in the channel (3) or a subsequent collection region (20).

20. Use according to claim 19, **characterized in that** the content of glucose, ketones or lactate is determined in the sample liquid (2), particularly in the blood plasma, by means of the chemical, and/or that the blood clotting is prevented by means of another chemical, particularly wherein fibrinogen present in the blood (19) is precipitated and filtered and/or separated by means of the other chemical and wherein the parameter is determined in the sample liquid (2) obtained.

21. Use according to claim 19 or 20, **characterized in that** the parameter or blood component is determined optically or electrochemically in the sample liquid (2), particularly directly in the channel (3) or in a collecting region (20) formed thereby.

## Revendications

1. Dispositif (1) pour recueillir du sang (19) et séparer des constituants sanguins, tels que du plasma sanguin, sous forme d'échantillon liquide (2), avec un dispositif d'acheminement (13) destiné au recueil du sang (19), un dispositif de séparation (15) destiné à la séparation de constituants sanguins sous forme d'échantillon liquide (2) et un canal (3) recueillant, de préférence exclusivement, l'échantillon liquide (2) sous l'effet de forces capillaires, une zone d'entrée (18) du canal (3) étant raccordée au dispositif de séparation (15) destiné au recueil de l'échantillon de liquide (2) séparé, le dispositif (1) présentant un dispositif de désaération afin de désaérer l'échantillon de liquide (2) dans la zone d'entrée (18) transversalement au sens principal de remplissage (F) et/ou au sens longitudinal du canal (3), et le dispositif (1) présentant un dispositif de remplissage pour remplir d'échantillon liquide (2) le canal (3) entre des faces ou côtés plats (8, 9) opposés délimitant le canal (3) dans la zone d'entrée (18),
**caractérisé en ce que**
le dispositif de remplissage présente une rampe (25) réduisant la hauteur (H) du canal (3) dans la zone d'entrée (18), et/ou **en ce que** la section du canal (3) dans la zone d'entrée (18) se rétrécit depuis le dispositif de séparation (15) vers le fond (8).

2. Dispositif selon la revendication 1, **caractérisé en ce que** le dispositif de désaération pour désaérer l'échantillon liquide (2) e$t aménagé directement sur le côté d'écoulement du dispositif de séparation (15), et/ou **en ce que** le dispositif de désaération pour désaérer l'échantillon liquide (2) est aménagé dans la zone d'entrée (18) sur le principal plan d'extension (E) du canal (3) et/ou le long d'un côté plat du dispositif de séparation (15) et/ou transversalement au sens d'écoulement de l'échantillon liquide (2) à travers le dispositif de séparation (15), et/ou **en ce que** le dispositif de désaération présente au moins un canal transversal (30) dans la zone d'entrée (18) transversal au principal sens de remplissage (F) du canal (3) d'échantillon liquide (2).

3. Dispositif selon l'une quelconque des revendications précédentes, **caractérisé en ce que** le dispositif de désaération présente un évidement (5) raccordé au canal (3) sur le côté.

4. Dispositif selon la revendication 3, **caractérisé en ce que** l'évidement (5) est raccordé au canal (3) à angles vifs afin de former un arrêt du liquide et/ou **en ce que** l'évidement (5) passe le long d'un principal sens de remplissage (F) d'échantillon liquide (2) et du canal (3) et/ou le long des petits et/ou longs côtés (4) ouverts du canal (3), de préférence opposés, et/ou **en ce que** l'évidement (5) est aménagé autour, en particulier entoure ou délimite le canal (3) de tous les côtés.

5. Dispositif selon la revendication 3 ou la revendication 4, **caractérisé en ce que** l'évidement (5) a une section au moins essentiellement rectangulaire et/ou **en ce que** l'évidement (5) est évidé sur un côté ou des deux par rapport à une projection latérale du canal (3), et/ou **en ce que** le dispositif (1) présente un support (6) et un recouvrement (7) entre lesquels ou à partir desquels le canal (3) et l'évidement (5) sont aménagés, et/ou **en ce que** le dispositif de désaération présente au moins un canal de désaération (12) raccordé à l'évidement (5).

6. Dispositif selon l'une quelconque des revendications précédentes, **caractérisé en ce que** l'échantillon de liquide (2) de la zone d'entrée (2) peut être recueilli verticalement par rapport au principal d'extension (E) du canal (3), en particulier verticalement à la position d'emploi, et/ou **en ce que** le dispositif de séparation (15) est plat, la zone d'entrée (18) étant alors raccordée directement à un côté plat du dispositif de séparation (15) et le principal sens de remplissage (F) du canal (3) étant en particulier parallèle au côté plat, et/ou **en ce que** le dispositif de séparation (15) présente un filtre ou une membrane pour séparer le plasma sanguin en tant qu'échantillon liquide (2).

7. Dispositif selon l'une quelconque des revendications précédentes, **caractérisé en ce que** le dispositif de remplissage présente au moins une structure (17) de préférence de type entretoise, palier, bosse ou colonne, qui dépasse d'une face ou d'un côté plat (8, 9).

8. Dispositif selon l'une quelconque des revendications précédentes, **caractérisé en ce que** la section du canal (3) se rétrécit essentiellement en forme de V à partir du dispositif de séparation (15) vers le fond (8).

9. Dispositif selon l'une quelconque des revendications précédentes, **caractérisé en ce que** le canal (3) présente plusieurs canaux individuels (27) du moins dans la zone d'entrée (18) et/ou n'est délimité ou formé, de préférence, que par deux faces ou côtés plats (8, 9) opposés, en particulier essentiellement plan(e)s.

10. Dispositif selon l'une quelconque des revendications précédentes, **caractérisé en ce que** le canal (3) est aménagé sur des petits ou longs côtés (4) opposés ou sur tous les petits ou longs côtés (4), en particulier latéralement autour, et ouvert et l'échantillon liquide (2) peut être guidé dans le canal (3) sans paroi latérale, et/ou en ce que le canal (3) a une section essentiellement rectangulaire et/ou plate, en particulier transversalement au principal sens de remplissage (F) de l'échantillon liquide (2) ou transversalement à l'extension longitudinale du canal (3).

11. Dispositif selon l'une quelconque des revendications précédentes, **caractérisé en ce que** la hauteur (H) du canal (3) est en particulier au moins 5 fois plus petite que la largeur (B) du canal (3), et/ou **en ce que** le volume disponible du canal (3) est inférieur à 1 ml, en particulier inférieur à 100 µl, de manière particulièrement préférée au maximum de 10 µl, et/ou **en ce que** le canal (3) forme au moins un réservoir pour l'échantillon liquide (2), et/ou **en ce que** le canal (3) contient un produit chimique, en particulier un produit chimique sec.

12. Dispositif selon l'une quelconque des revendications précédentes, **caractérisé en ce que** le canal (3) présente au moins un élément de guidage pour agir sur, en particulier homogénéiser, le remplissage avec l'échantillon liquide (2) et/ou **en ce que** le canal (3) présente des aspérités (10), réparties de préférence régulièrement, en tant qu'éléments de guidage, disposées en particulier en lignes transversalement ou parallèlement au principal sens de remplissage (F).

13. Dispositif selon la revendication 12, **caractérisé en ce que** les aspérités (10) sont en forme de bosses ou de colonnes, en particulier ont une base ronde ou polygonale, et/ou **en ce que** la densité superficielle, la distance et/ou la taille des éléments de guidage varie, en particulier en fonction de la distance de chacune par rapport à une entrée destinée à l'échantillon liquide (2) dans le canal (3).

14. Dispositif selon la revendication 12 ou la revendication 13, **caractérisé en ce que** le canal (3) présente au moins une rigole (11) en tant qu'élément de guidage qui est en particulier transversale ou parallèle au principal sens de remplissage (F) du canal (3) et forme de préférence un arrêt uniquement temporaire du liquide pour homogénéiser le front (V) de l'échantillon de liquide (2), en particulier la rigole (11) ayant une section au moins essentiellement rectangulaire ou semi-circulaire et/ou sa profondeur étant plus petite que la profondeur de l'évidement (5).

15. Dispositif selon l'une quelconque des revendications précédentes, **caractérisé en ce que** le dispositif d'acheminement (13) et/ou le dispositif de séparation (15) présente un premier produit chimique, en particulier un agent pour la lyse de cellules, de préférence de cellules sanguines, en particulier le premier produit chimique se présentant sous forme de produit chimique sec et pouvant être dissous par le liquide acheminé, tel que l'échantillon liquide (2) et/ou le sang (19).

16. Dispositif selon l'une quelconque des revendications précédentes, **caractérisé en ce que** le canal (3), en particulier sa zone de collecte (20), est pourvu d'au moins un deuxième produit chimique, en particulier le deuxième produit chimique se présentant sous forme de produit chimique sec et pouvant être dissous par l'échantillon liquide (2), en particulier le plasma sanguin et/ou le liquide intracellulaire, et/ou en particulier le deuxième produit chimique permettant la détermination de la teneur en Ca, et/ou contenant un agent complexant, en particulier de la o-crésolphtaléine, pour former un complexe coloré.

17. Dispositif selon l'une quelconque des revendications précédentes, **caractérisé en ce que** seul un canal unique (3) pour recueillir et/ou dévier l'échantillon liquide (2) est raccordé au dispositif de séparation (15).

18. Dispositif selon l'une quelconque des revendications précédentes, **caractérisé en ce que** le dispositif (1) présente un détecteur (24) disposé de préférence sur un côté plat (9) du canal (3) pour mesurer la lumière diffusée ou la fluorescence, et/ou en ce que le dispositif (1) présente un dispositif de prélèvement d'un échantillon liquide (2).

19. Utilisation d'un dispositif (1) selon l'une quelconque des revendications 1 à 18 pour déterminer au moins un élément constituant dans le sang (19) ou pour analyser le plasma sanguin, en particulier des protéines ou des électrolytes, en tant qu'échantillon liquide (2), l'échantillon liquide (2) étant acheminé et/ou filtré de préférence exclusivement sous l'effet de forces capillaires du canal (3) et un ou plusieurs constituants et/ou paramètres étant déterminé(s) au moyen d'un produit chimique, de préférence directement dans le canal (3) ou dans une zone de collecte (20) raccordée.

20. Utilisation selon la revendication 19, **caractérisée en ce qu'**on détermine la teneur en glucose, en cétones ou en lactate dans l'échantillon liquide (2), en particulier dans le plasma sanguin, au moyen du produit chimique, et/ou **en ce que** la coagulation du sang est empêchée au moyen d'un autre produit chimique, en particulier le fibrinogène présent dans le sang étant en particulier précipité et filtré ou séparé au moyen de l'autre produit chimique et le paramètre déterminé dans l'échantillon liquide (2) qui se forme.

21. Utilisation selon l'une quelconque des revendications 19 ou 20, **caractérisée en ce que** le paramètre ou le constituant du sang (19) est déterminé visuellement ou par voie électrochimique dans l'échantillon liquide (2), en particulier directement dans le canal (3) ou dans une zone de collecte (20) formée par celui-ci.
